(19) Europäisches Patentamt
European Patent Office
Office européen des brevets

(11) Numéro de publication : **0 119 107**
**B1**

(12) # FASCICULE DE BREVET EUROPÉEN

(45) Date de publication du fascicule du brevet : **01.10.86**

(21) Numéro de dépôt : **84400056.2**

(22) Date de dépôt : **12.01.84**

(51) Int. Cl.⁴ : **C 07 D233/06**, C 07 D233/20, C 07 D233/22, C 07 D239/06, C 07 D405/04, A 61 K 31/415, A 61 K 31/505

(54) **Nouveaux dérivés du bicyclo(4.2.0)octatriène-1,3,5, leur préparation et leur application en thérapeutique.**

(30) Priorité : **14.01.83 FR 8300502**

(43) Date de publication de la demande : **19.09.84 Bulletin 84/38**

(45) Mention de la délivrance du brevet : **01.10.86 Bulletin 86/40**

(84) Etats contractants désignés : **AT BE CH DE FR GB IT LI LU NL SE**

(56) Documents cités :
**US-A- 2 948 749**
**US-A- 3 287 469**

(73) Titulaire : **ADIR**
**22, rue Garnier**
**F-92200 Neuilly-sur-Seine (FR)**

(72) Inventeur : **Malen, Charles**
**3 allée Traversière**
**F-94260 Fresnes (FR)**
Inventeur : **Peglion, Jean-Louis**
**33 Avenue De Verdun**
**F-78400 Chatou (FR)**
Inventeur : **Laubie, Michel**
**35 Avenue Foch**
**F-92420 Vaucresson (FR)**
Inventeur : **Poignant, Jean-Claude**
**Rue de la Fontaine St-Mathieu**
**F-91440 Bures S/Yvette (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 119 107 B1

Jouve, 18, rue St-Denis, 75001 Paris, France

**Description**

La présente invention a pour objet de nouveaux dérivés du bicyclo [4.2.0] octatriène-1,3,5, des méthodes pour les préparer et les compositions pharmaceutiques les contenant.

Les composés de l'invention répondent à la formule générale I :

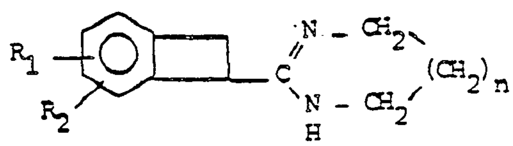

(I)

dans laquelle

$R_1$ et $R_2$ représentent chacun, indépendamment l'un de l'autre, un atome d'hydrogène, un atome d'halogène, un radical alkyle ou alcoxy ayant de 1 à 4 atomes de carbone, un radical sulfonamide éventuellement substitué par un reste alkyle ayant de 1 à 4 atomes de carbone, ou bien ensemble un radical méthylènedioxy,

n représente un nombre entier égal à 0 ou 1,

sous forme racémique ou d'isomères optiques, ainsi que leurs sels d'addition à des acides pharmaceutiquement acceptables.

Les composés selon l'invention possèdent un atome de carbone asymétrique et peuvent donc être dédoublés en leurs isomères optiques. Les sels d'addition sont obtenus avec des acides minéraux ou organiques pharmaceutiquement acceptables tels que l'acide chlorhydrique, sulfurique, méthanesulfonique, tartrique, citrique, fumarique, maléïque, etc...

Parmi les composés de l'invention, on préfère ceux qui répondent à la formule I dans laquelle $R_1$ et $R_2$ représentent chacun un atome d'hydrogène.

L'invention s'étend également à leur mode d'obtention caractérisé en ce que l'on condense en présence d'$H_2S$ un dérivé de formule générale II :

$$H_2N-CH_2-(CH_2)_n-CH_2-NH_2 \qquad (II)$$

dans laquelle n a la même signification que dans la formule I, avec un dérivé de formule générale III :

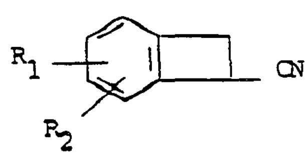

(III)

dans laquelle $R_1$ et $R_2$ ont les mêmes significations que dans la formule I.

Cette réaction pourra être avantageusement conduite à température ambiante ou par chauffage modéré, et, éventuellement, dans un solvant polaire tel que le méthanol, l'éthanol, le propanol.

Les isomères optiques des composés de l'invention peuvent être séparés de manière classique après salification par un acide optiquement actif tel que l'acide tartrique, et cristallisation fractionnée des sels, puis si on le désire, libération de la base optiquement active par une base forte ou transsalification par un acide fort. Cependant, les bases optiquement actives pevuent se racémiser facilement et il est préférable de conserver les énantiomères sous forme de leurs sels d'addition, généralement stables à l'état solide.

Les composés de formule générale III sont généralement connus dans la littérature, et par contre, ceux pour lesquels $R_1$ représente un atome d'hydrogène et $R_2$ représente un atome de chlore en position — 2 ou — 3, un atome de fluor en position — 4 ou un radical sulfonamide en position — 4 sont nouveaux.

Certains bicyclooctatriènes (tétrahydrozoline) substitués par un groupement imidazolinyl-2 sont connus dans la littérature (brevets U.S. 2,731,471 de Sahyun et U.S. 2,842,478 de Pfizer) et possèdent une activité agoniste α-adrénergique qui justifie en particulier leur emploi en thérapeutique comme vasoconstricteurs et décongestionnants nasaux.

Les composés de l'invention possèdent une structure bicyclo [4.2.0] octatriène-1,3,5-ique substituée par un groupement imidazolinyl-2, mais ils présentent des effets totalement opposés, puisqu'ils sont antagonistes α-adrénergiques.

D'autres dérivés hétérocycliques substitués par une imidazoline sont connus dans la littérature (brevet EP 71.368 Reckitt et Colmann) et présentent également une activité antagoniste α-adrénergique.

Mais les composés de l'invention s'en distinguent notamment par la nature de leur cycle et par la sélectivité de leurs isomères sur les récepteurs $\alpha_1$ ou $\alpha_2$, ce qui permet de les utiliser en thérapeutique dans des indications différentes.

Les exemples suivants illustrent la préparation des composés de l'invention. Les températures de fusion (F °C) ont été mesurées sur bloc « Micro-Kofler ».

## Exemple 1

(d, l) (imidazolinyl-2)-7 bicyclo [4.2.0] octatriène-1,3,5

Dans un récipient on introduit successivement 5 grammes de cyano-7 bicyclo [4.2.0] octatriène-1,3,5 (préparé selon la méthode décrite dans Org. Synth. Coll. Vol. V p. 263), puis 7,2 grammes de diamino-1,2 éthane et on sature le mélange en $H_2S$ tout en le maintenant à l'abri de l'humidité. Après quatre jours de contact sous agitation intermittente de la masse pâteuse ainsi formée, on ajoute 30 $cm^3$ d'eau, on alcalinise par de la soude et on extrait la solution par du chlorure de méthylène qui est lavé à l'eau, séché et évaporé. L'(imidazolinyl-2)-7 bicyclo [4.2.0] octatriène-1,3,5 racémique est obtenu soit sous forme de base après distillation du résidu obtenu précédemment à 120 °C sous 0,02 mm/Hg et recristallisation dans l'acétate d'éthyle, soit sous forme de chlorhydrate.

F °C. 102-107 (base) ; 180-190 (chlorhydrate) avec décomposition.

Caractéristiques spectrales en :

IR : bande NH : 3 220 $cm^{-1}$
bande C = N : 1 610 $cm^{-1}$
RMN : 3,4 ppm (2H) ; 3,5 ppm (4H) ;
6,2 ppm (1H échangeable)
6,3 ppm (1H) ; 7,2 ppm (4H aromatiques)

## Exemple 2

Tartrate (—) d'(imidazolinyl-2)-7 bicyclo [4.2.0] octatriène-1,3,5.

On porte à ébullition 8 grammes d'(imidazolinyl-2)-7 bicyclo [4.2.0] octatriène-1,3,5 obtenu dans l'exemple 1 et 6,9 grammes d'acide tartrique (+) en solution dans 30 $cm^3$ d'éthanol. On reprend le résidu par 50 $cm^3$ d'isopropanol et on évapore à sec. Après cinq recristallisations successives, on obtient le tartrate (—) d'(imidazolinyl-2)-7 bicyclo [4.2.0] octatriène-1,3,5.

F °C : 155-160

$[\alpha]^{22°}_{589} = -61{,}7$ $[\alpha]^{22°}_{365} = -218$ (0,5 % $H_2O$)

## Exemple 3

Tartrate (+) d'(imidazolinyl-2)-7 bicyclo [4.2.0] octatriène-1,3,5.

En remplaçant dans l'exemple 2 l'acide tartrique (+) par l'acide tartrique (—), on obtient de la même façon le tartrate (+) d'(imidazolinyl-2)-7 bicyclo [4.2.0] octatriène-1,3,5.

F °C : 155-160

## Exemple 4

Méthoxy-3 (imidazolinyl-2)-7 bicyclo [4.2.0] octatriène-1,3,5.

On dissout 9,6 grammes de méthoxy-3 cyano-7 bicyclo [4.2.0] octatriène-1,3,5 (préparé selon le mode opératoire décrit dans J. Am. Chem. Soc. 98 (11) 3378-9 (1976) dans 10,8 grammes de diamino-1,2 éthane. On sature la solution en $H_2S$ en maintenant la température à 0 °C et on l'abandonne une nuit à température ambiante, puis on chauffe le mélange pendant 18 heures à 50 °C. Après refroidissement, on ajoute de la soude diluée et on extrait par du chlorure de méthylène. La phase organique est prélevée et extraite par une solution d'acide chlorhydrique 1N. La phase aqueuse est à nouveau extraite par du chlorure de méthylène qui est séché et évaporé sous pression réduite. Le résidu obtenu est purifié par traitement à température ambiante par de l'anhydride acétique dans l'acide acétique, puis, dilution par de l'eau, alcalinisation par de la lessive de soude et extraction par du chlorure de méthylène. La phase organique est lavée à l'eau, séchée et évaporée sous pression réduite. Après recristallisation dans l'acétonitrile, on obtient 6,6 grammes de méthoxy-3 (imidazolinyl-2)-7 bicyclo [4.2.0] octatriène-1,3,5 qui peut être transformé en son fumarate.

F °C : 128-132 (base) ; 135-139 (fumarate)

Analyse centésimale (fumarate)

| | | | |
|---|---|---|---|
| Calculé | C% 60,37 | H% 5,70 | N% 8,80 |
| Trouvé | C% 60,29 | H% 5,69 | N% 8,89 |

pour $C_{12}H_{14}N_2O$, $C_4H_4O_4$ = 318,33

Exemple 5

Méthoxy-4 (imidazolinyl-2)-7 bicyclo [4.2.0] octatriène-1,3,5.

En remplaçant dans l'exemple 4 le méthoxy-3 cyano-7 bicyclo [4.2.0] octatriène-1,3,5 par le méthoxy-4 cyano-7 bicyclo [4.2.0] octatriène-1,3,5 préparé selon le mode opératoire décrit dans Tetrahedron 30, 1053 (1974), on obtient le méthoxy-4 (imidazolinyl-2)-7 bicyclo [4.2.0] octatriène-1,3,5.

F °C : 117-120 (base) ; 178-180 (fumarate)

Analyse centésimale (fumarate)

| | | | |
|---|---|---|---|
| Calculé | C% 60,37 | H% 5,70 | N% 8,80 |
| Trouvé | C% 60,00 | H% 5,66 | N% 8,64 |

pour $C_{12}H_{14}N_2O$, $C_4H_4O_4$ = 318,33

Exemple 6

Chloro-4 (imidazolinyl-2)-7 bicyclo [4.2.0] octatriène-1,3,5.

En remplaçant dans l'exemple 4 le méthoxy-3 cyano-7 bicyclo [4.2.0] octatriène-1,3,5 par le chloro-4 cyano-7 bicyclo [4.2.0] octatriène-1,3,5 préparé selon le mode opératoire décrit dans J. Am. Chem. Soc. 33 (8), 3327-9 (1968), on obtient le chloro-4 (imidazolinyl-2)-7 bicyclo [4.2.0] octatriène-1,3,5.

F °C : 127-130 (base), 171-174 (fumarate)

Analyse centésimale (fumarate)

| | | | |
|---|---|---|---|
| Calculé | C% 55,82 | H% 4,68 | N% 8,68 |
| Trouvé | C% 55,73 | H% 4,74 | N% 8,63 |

pour $C_{11}H_{11}ClN_2$, $C_4H_4O_4$ = 322,75

Exemple 7

Diméthoxy-3,4 (imidazolinyl-2)-7 bicyclo [4.2.0] octatriène-1,3,5.

En remplaçant dans l'exemple 4 le méthoxy-3 cyano-7 bicyclo [4.2.0] octatriène-1,3,5 par le diméthoxy-3,4 cyano-7 bicyclo [4.2.0] octatriène-1,3,5 préparé selon le mode opératoire décrit dans Tetrahedron 29, 73 (1973), on obtient le diméthoxy-3,4 (imidazolinyl-2)-7 bicyclo [4.2.0] octatriène-1,3,5.

F °C : 158-161 (base) ; 215 (fumarate)

Analyse centésimale (fumarate)

| | | | |
|---|---|---|---|
| Calculé | C% 58,61 | H% 5,79 | N% 8,04 |
| Trouvé | C% 58,52 | H% 5,79 | N% 7,94 |

pour $C_{13}H_{16}N_2O_2$, $C_4H_4O_4$ = 348,35

Exemple 8

Chloro-2 (imidazolinyl-2)-7 bicyclo [4.2.0] octatriène-1,3,5.

Stade A

Acide cyano-2 (dichloro-2,6 phényl)-3 propanoïque.

On met en suspension 92 grammes d'acide cyano-2 (dichloro-2,6 phényl)-3 propénoïque (obtenu selon le mode opératoire décrit dans Tetrahedron Lett. 1975, 3643-4) dans 860 $cm^3$ de méthanol et 285 $cm^3$ d'une solution à 10 % de carbonate acide de sodium. On refroidit à 18 °C et on introduit en 2 heures 30 la quantité nécessaire de borohydrure de sodium. On abandonne le mélange pendant 2 heures à température ambiante, on concentre et on acidifie jusqu'à pH 2,3 par de l'acide chlorhydrique 1N. On extrait la solution à l'éther que l'on lave à l'eau, sèche sur sulfate de magnésium, filtre et évapore. On obtient 82,4 g d'acide cyano-2 (dichloro-2,6 phényl)-3 propanoïque.

F °C : 106-108

Stade B

(dichloro-2,6 phényl)-3 propionitrile

On dissout dans 140 $cm^3$ de N,N diméthylaminoacétamide 81,3 grammes d'acide cyano-2 (dichloro-2,6 phényl)-3 propanoïque obtenu précédemment et l'on chauffe pendant 1 heure à 140-150 °C. Après refroidissement, on verse le milieu réactionnel dans 600 $cm^3$ d'eau, on extrait par 3 200 $cm^3$ d'éther que l'on lave avec une solution saturée de carbonate acide de sodium, puis par une solution saturée de chlorure de sodium. La phase éthérée est décolorée, séchée et évaporée. On obtient 61,7 grammes de (dichloro-2,6 phényl)-3 propionitrile sous forme d'une huile qui cristallise à température ambiante.

Stade C

Chloro-2 cyano-7 bicyclo [4.2.0] octatriène-1,3,5.

En appliquant le mode opératoire décrit dans Org. Synth. Coll. Vol. V p. 263, à 20 grammes de (dichloro-2,6 phényl)-3 propionitrile obtenu précédemment, on obtient, après distillation à 114-118 °C sous 0,05 mm Hg, 10,4 grammes de chloro-2 cyano-7 bicyclo [4.2.0] octatriène-1,3,5.

Caractéristique spectrale en IR : bande C = N à 2 240 $cm^{-1}$.

Stade D

Chloro-2 (imidazolinyl-2)-7 bicyclo [4.2.0] octatriène-1,3,5.

En remplaçant dans l'exemple 4 le méthoxy-3 cyano-7 bicyclo [4.2.0] octatriène-1,3,5 par le chloro-2 cyano-7 bicyclo [4.2.0] octatriène-1,3,5 obtenu au stade précédent, on obtient le chloro-2 (imidazolinyl-2)-7 bicyclo [4.2.0] octatriène-1,3,5.

F °C : 100-106 (base) ; 189-196 (fumarate)

Analyse centésimale (fumarate)

| | | | |
|---|---|---|---|
| Calculée | C% 55,82 | H% 4,68 | N% 8,68 |
| Trouvé | C% 55,76 | H% 4,78 | N% 8,70 |

pour $C_{11}H_{11}ClN_2$, $C_4H_4O_4$ = 322,75

## Exemple 9

Chloro-2 (tétrahydropyrimidinyl-2)-7 bicyclo [4.2.0] octatriène-1,3,5.

En remplaçant dans l'exemple 4 le diamino-1,2 éthane par le diamino 1,3 propane, et le méthoxy-3 cyano-7 bicyclo [4.2.0] octatriène-1,3,5 par le chloro-2 cyano-7 bicyclo [4.2.0] octatriène-1,3,5 (exemple 8 stade C), on obtient le chloro-2 (tétrahydropyrimidinyl-2)-7 bicyclo [4.2.0] octatriène-1,3,5.

F °C : 133-136 (base) ; 185-195 (fumarate)

Analyse centésimale (fumarate)

| | | | |
|---|---|---|---|
| Calculé | C% 57,06 | H% 5,09 | N% 8,32 |
| Trouvé | C% 56,81 | H% 5,09 | N% 8,32 |

pour $C_{12}H_{13}ClN_2$, $C_4H_4O_4$ = 336,77

## Exemple 10

Chloro-3 (imidazolinyl-2)-7 bicyclo [4.2.0] octatriène-1,3,5.

Stade A

nitro-3 cyano-7 bicyclo [4.2.0] octatriène-1,3,5.

La réaction de nitration du cyano-7 bicyclo [4.2.0] octatriène-1,3,5 décrite dans J. Org. Chem. 33 (8) 3327 (1968) conduit au nitro-4 cyano-7 bicyclo [4.2.0] octatriène-1,3,5. Toutefois, une faible proportion du dérivé nitré en position 3 peut-être obtenue. Ainsi, à partir de 77,4 g de cyano-7 bicyclo [4.2.0] octatriène-1,3,5 on obtient, après chromatographie sur des eaux-mères de recristallisation du dérivé nitré en position —4, sur colonne de silice (Kieselgel 60,70.230 mesh ASTM) en éluant par un mélange de cyclohexane-acétate d'éthyle (70 : 30), 3,3 grammes de nitro-3 cyano-7 bicyclo [4.2.0] octatriène-1,3,5 qui est recristallisé dans l'éthanol absolu (F °C 73-77).

Stade B

Amino-3 cyano-7 bicyclo [4.2.0] octatriène-1,3,5.

La réduction sur Pd/C à 5 % dans l'éthanol en présence d'acide acétique de 4,7 grammes de nitro-3 cyano-7 bicyclo [4.2.0] octatriène-1,3,5 obtenu au stade précédent conduit à 3,3 grammes d'amino-3 cyano-7 bicyclo [4.2.0] octatriène-1,3,5.

F °C chlorhydrate : 195 (décomposition)

Stade C

Chloro-3 cyano-7 bicyclo [4.2.0] octatriène-1,3,5.

Par réaction de Sandmeyer, on obtient à partir de 3,2 grammes d'amino-3 cyano-7 bicyclo [4.2.0] octatriène-1,3,5 obtenu au stade précédent une huile qui après distillation à 100 °C sous 0,05 mm Hg donne 2,1 grammes de chloro-3 cyano-7 bicyclo [4.2.0] octatriène-1,3,5 ($n^{24}_D$ : 1,563 9)

Stade D

Chloro-3 (imidazolinyl-2)-7 bicyclo [4.2.0] octatriène-1,3,5.

Dans 12 $cm^3$ de méthanol anhydre on mélange 3,7 grammes de diamino-1,2 éthane et 2 grammes de chloro-3 cyano-7 bicyclo [4.2.0] octatriène-1,3,5 obtenu au stade précédent, on sature d'$H_2S$ en maintenant la température à 0 °C puis on porte à ébullition pendant 4 heures. Après évaporation du solvant le résidu est repris par une solution diluée de soude qui est extraite par du chlorure de méthylène. Après extraction de la phase organique par une solution d'acide chlorhydrique 1N, relargage de cette phase acide par de la lessive de soude et extraction par du chlorure de méthylène, on obtient 2,1 grammes de chloro-3 (imidazolinyl-2)-7 bicyclo [4.2.0] octatriène-1,3,5 qui est recristallisé dans l'acétonitrile.

F °C : 127-133 (base) ; 149-152 (fumarate)

Analyse centésimale (fumarate)

| | | | |
|---|---|---|---|
| Calculé | C% 55,82 | H% 4,68 | N% 8,68 |
| Trouvé | C% 55,55 | H% 4,62 | N% 8,57 |

pour $C_{11}H_{11}ClN_2$, $C_4H_4O_4$ = 322,75

## Exemple 11

Fluoro-4 (imidazolinyl-2)-7 bicyclo [4.2.0] octatriène-1,3,5.

Stade A

Fluoro-4 cyano-7 bicyclo [4.2.0] octatriène-1,3,5.

En appliquant les conditions de la réaction de SCHIEMANN à 14,4 grammes d'amino-4 cyano-7 bicyclo [4.2.0] octatriène-1,3,5 préparé selon le mode opératoire décrit dans J. Org. Chem, 33 (8), 3327 (1968), on obtient 3,2 grammes d'une huile brune qui, par distillation à 125 °C sous 0,05 mm Hg, conduit à 1,4 grammes de fluoro-4 cyano-7 bicyclo [4.2.0] octatriène-1,3,5 ($n^{25}_D$ = 1,508 2)

Stade B

Fluoro-4 (imidazolinyl-2)-7 bicyclo [4.2.0] octatriène-1,3,5.

En remplaçant dans l'exemple 10 stade D le chloro-3 cyano-7 bicyclo [4.2.0] octatriène-1,3,5 par le fluoro-4 cyano-7 bicyclo [4.2.0] octatriène-1,3,5, on obtient le fluoro-4 (imidazolinyl-2)-7 bicyclo [4.2.0] octatriène-1,3,5.

F °C : 98-103 (base) ; 181-185 (fumarate)

Analyse centésimale (fumarate)

| | | | |
|---|---|---|---|
| Calculé | C% 58,82 | H% 4,93 | N% 9,14 |
| Trouvé | C% 58,51 | H% 4,93 | N% 9,03 |

## Exemple 12

Sulfamoyl-4 (imidazolinyl-2)-7 bicyclo [4.2.0] octatriène-1,3,5.

Stade A

Sulfamoyl-4 cyano-7 bicyclo [4.2.0] octatriène-1,3,5.

L'application des conditions de la réaction de MEERWEIN (diazotation par $NaNO_2$ dans l'acide chlorhydrique concentré, puis traitement par une suspension de $CuCl_2$ dans une solution acétique de $SO_2$) à 10 grammes d'amino-4 cyano-7 bicyclo [4.2.0] octatriène-1,3,5 permet l'obtention du sulfochlorure correspondant, puis traité immédiatement par une solution concentré d'ammoniaque, conduit à 2,2 grammes de sulfamoyl-4 cyano-7 bicyclo [4.2.0] octatriène-1,3,5 (recristallisé du méthanol).

F °C : 200-203.

Stade B

Sulfamoyl-4 (imidazolinyl-2)-7 bicyclo [4.2.0] octatriène-1,3,5.

A 10 $cm^3$ de méthanol on ajoute 2,9 grammes de diamino-1, 2 éthane et 2 grammes de sulfamoyl-4 cyano-7 bicyclo [4.2.0] octatriène-1,3,5 obtenu au stade prédédent, on sature en $H_2S$ tout en maintenant la température à 0°C et on porte à ébullition pendant 4 heures. Par évaporation du solvant on obtient 3 grammes d'une huile brune qui cristallise dans 5 $cm^3$ d'acétonitrile. Après recristallisation dans l'éthanol, on obtient 1,1 g de sulfamoyl-4 (imidazolinyl-2)-7 bicyclo [4.2.0] octatriène-1,3,5.

F °C : 200 (décomposition)

Caractéristiques spectrales en infra-rouge :

bandes $NH_2$ : 3 300 et 3 380 $cm^{-1}$, 1 620 $cm^{-1}$
bandes NH : 3 100 à 2 000 $cm^{-1}$
bandes $SO_2$ : 1 160 et 1 330 $cm^{-1}$

Etude pharmacologique des composés de l'invention

Les composés de l'invention ont été testés sur les α-adrénorécepteurs périphériques et centraux, vis-à-vis de réactifs classiques : la cirazoline (agoniste $\alpha_1$), l'azépexole (agoniste $\alpha_2$), la clonidine (agoniste $\alpha_1$ et $\alpha_2$) et le « ST587 » (agoniste $\alpha_1$), sur les récepteurs périphériques (« vas deferens ») et les récepteurs centraux (sommeil du poussin).

A titre d'exemple, chez le rat amyélé, le composé de l'exemple I ainsi que ses isomères, administrés à la dose de 1 mg/kg IV, concurrencent de manière significative l'effet de la cirazoline, agoniste $\alpha_1$ assez spécifique (Lefevre et coll. Eur. J. pharmacol. 43, 85 (1977). Le même type d'expérience a été réalisée avec le « ST587 », à savoir la (chloro-2 trifluorométhyl-5 phenyl-imino)-2 imidazolidine, agoniste $\alpha_1$ sélectif (De Jong et coll., Life Science, Vol. 28, ; 203-206 (1982). Ces résultats montrent que les composés de l'invention sont actifs pour antagoniser les adrénorécepteurs $\alpha_1$ postjonctionnels du rat.

L'étude vis-à-vis d'un agoniste $\alpha_2$ sélectif, l'azépéxole ou amino-2 éthyl-6 tétrahydro-5,6,7,8-(4 H) oxazolo- [4,5-d] -azépine, dichlorhydrate (Timmerman et Van Zwieten, Eur. J. Pharmacol. 63, 199-202 (1980)) ; a montré que les composés de l'invention sont très actifs à la dose de 1 mg/kg IV chez le rat amyélé.

Les résultats obtenus en comparaison avec la clonidine, sur les deux types d'adrénorécepteurs (plus spécifiquement sur $\alpha_2$) sont en accord avec les études précédentes. Chez le poussin, les composés de l'invention à la dose de 1 mg/kg IV provoquent l'inhibition du sommeil induit par la clonidine à la dose de 0,750 mg/kg.

Finalement, on a constaté chez le chien anesthésié au nembutal, soumis à la respiration artificielle, que les composés inversent les effets tensionnels de l'adrénaline et réduisent ceux de la noradrénaline.

Les expériences précédentes montrent que les composés de l'invention sont actifs sur les adréno-récepteurs $\alpha_1$ et $\alpha_2$, les isomères étant plus particulièrement sélectifs sur l'un ou l'autre de ces récepteurs.

Par conséquent, ils peuvent être utilisés en thérapeutique, en particulier, dans le traitement de l'hypertension, de la dépression, de l'obésité, de l'asthme ou du diabète.

Les composés de l'invention peuvent être administrés par voie orale ou parentérale à la dose quotidienne de 0,1 à 1 mg/kg. Les compositions administrables par voie orale (comprimés, dragées, gélules,...) peuvent contenir par exemple de 5 à 50 mg de substance active, ainsi que les excipients pharmaceutiques habituels tels que le lactose, l'amidon, le talc... Les compositions destinées pour la voie parentérale contiendront de préférence une dose unitaire de 5 à 25 mg de substance active sous forme d'un sel d'addition en solution aqueuse.

Par exemple, les compositions peuvent être présentées sous forme de gélules contenant 10 mg de tartrate du composé de l'exemple 1 et 40 mg de lactose.

**Revendications** (pour les Etats contractants : BE, CH, DE, FR, GB, IT, LI, LU, NL, SE)

1. Composés répondant à la formule générale :

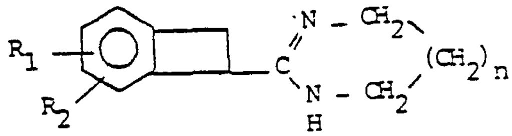

(I)

dans laquelle

$R_1$ et $R_2$ représentent chacun un atome d'hydrogène, un atome d'halogène, un radical alkyle ou alcoxy ayant de 1 à 4 atomes de carbone, un radical sulfonamide éventuellement substitué par un reste alkyle ayant de 1 à 4 atomes de carbone, ou bien ensemble un radical méthylène-dioxy,

n représente un nombre entier égal à 0 ou 1

sous forme racémique ou d'isomères optiques, ainsi que leurs sels d'addition à des acides pharmaceutiquement acceptables.

2. Composés selon la revendication 1, répondant à la formule générale I dans laquelle $R_1$ et $R_2$ représentent chacun un atome d'hydrogène.

3. Le (d,l) (imidazolinyl-2)-7 bicyclo [4.2.0] octatriène-1,3,5, ses isomères (d) et (1), ainsi que leurs sels d'addition à des acides pharmaceutiquement acceptables.

4. Procédé de préparation des composés selon la revendication I, procédé caractérisé en ce que l'on condense en présence d'$H_2S$ un dérivé de formule générale,

$$H_2N{-}CH_2{-}(CH_2)_n{-}CH_2{-}NH_2 \qquad \text{(II)}$$

dans laquelle n a la même signification que dans la formule I avec un dérivé de formule

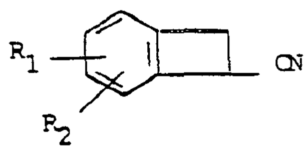

(III)

dans laquelle $R_1$ et $R_2$ ont les mêmes significations que dans la formule I à la température ambiante ou par chauffage modéré et éventuellement dans un solvant polaire.

5. Composition pharmaceutique contenant comme principe actif un composé selon l'une quelconque des revendications 1 à 3 et un excipient ou un véhicule pharmaceutiquement compatible.

**Revendications** (pour l'Etat contractant AT)

1. Procédé de préparation de dérivés du bicyclo [4.2.0] octatriène-1,3,5 répondant à la formule générale I :

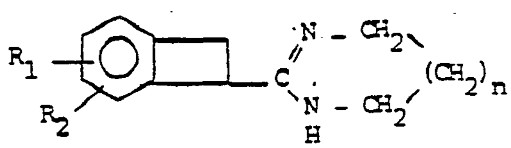

(I)

dans laquelle

$R_1$ et $R_2$ représentent chacun, indépendamment l'un de l'autre, un atome d'hydrogène, un atome d'halogène, un radical alkyle ou alcoxy ayant de 1 à 4 atomes de carbone, un radical sulfonamide éventuellement substitué par un reste alkyle ayant de 1 à 4 atomes de carbone, ou bien ensemble un radical méthylènedioxy,

n représente un nombre entier égal à 0 ou 1,

sous forme racémique ou d'isomères optiques, caractérisé en ce que l'on condense en présence d'$H_2S$ un dérivé de formule générale II :

$$H_2N{-}CH_2{-}(CH_2)n{-}CH_2{-}NH_2 \qquad \text{(II)}$$

dans laquelle n a la même signification que dans la formule I, avec un dérivé de formule générale III :

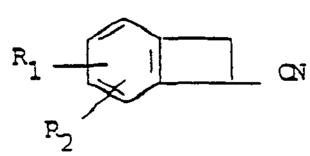

(III)

dans laquelle $R_1$ et $R_2$ ont les mêmes significations que dans la formule I.

2. Procédé selon la revendication 1 caractérisé en ce que cette réaction pourra être avantageusement conduite à température ambiante ou par chauffage modéré, et, éventuellement, dans un solvant polaire tel que le méthanol, l'éthanol, le propanol.

3. Procédé selon la revendication 1 caractérisé en ce que les isomères optiques des composés de la formule générale I peuvent être séparés après salification par un acide optiquement actif tel que l'acide tartrique, et cristallisation fractionnée des sels, puis si on le désire, libération de la base optiquement active par une base forte ou transsalification par un acide fort.

**Claims** (for the Contracting States : BE, CH, DE, FR, GB, IT, LI, LU, NL, SE)

1. Compounds corresponding to the general formula

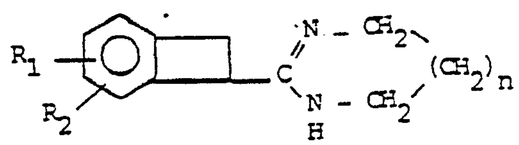

(I)

in which each of

$R_1$ and $R_2$ represents a hydrogen atom, a halogen atom, an alkyl or alkoxy radical having from 1 to 4 carbon atoms, or a sulphonamide radical optionally substituted by an alkyl radical having from 1 to 4 carbon atoms, or they together represent a methylenedioxy radical, and

n represents the integer 0 or 1,

in racemic form or in the form of optical isomers, and also their pharmaceutically acceptable acid addition salts.

2. Compounds according to claim 1, corresponding to the general formula I in which each of $R_1$ and $R_2$ represents a hydrogen atom.

3. dl-7-(2-Imidazolinyl)-bicyclo [4.2.0] octa-1,3,5-triene, its d and l isomers, and also pharmaceutically acceptable acid addition salts thereof.

4. Process for the preparation of the compounds according to claim 1, characterised in that, in the presence of $H_2S$, a derivative of the general formula

$$H_2N—CH_2—(CH_2)n—CH_2—NH_2 \quad (II)$$

in which n has the same meaning as in formula I, is condensed with a derivative of the formula

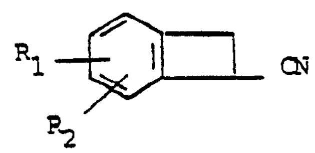

(III)

in which $R_1$ and $R_2$ have the same meanings as in formula I at ambient temperature or with moderate heating and, optionally, in a polar solvent.

5. Pharmaceutical composition containing as active ingredient a compound according to any one of claims 1 to 3 and a phyarmaceutically compatible excipient or carrier.

**Claims** (for the Contracting State AT)

1. Process for the preparation of bicyclo [4.2.0] octa-1,3,5-triene derivatives corresponding to the general formula I :

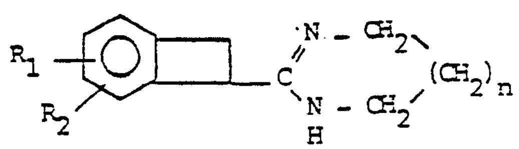

(I)

in which each of

$R_1$ and $R_2$ represents, independently of the other, a hydrogen atom, a halogen atom, an alkyl or alkoxy radical having from 1 to 4 carbon atoms, or a sulphonamide radical optionally substituted by an alkyl radical having from 1 to 4 carbon atoms, or they together represent a methylenedioxy radical, and

n represents the integer 0 or 1,

in racemic form or in the form of optical isomers, caracterised in that, in the presence of $H_2S$, a derivative of the general formula II :

$$H_2N—CH_2—(CH_2)n—CH_2—NH_2 \quad (II)$$

in which n has the same meaning as in formula I, is condensed with a derivative of the general formula III :

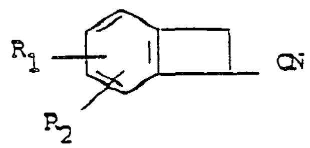

(III)

in which $R_1$ and $R_2$ have the same meanings as in formula I.

2. Process according to claim 1, characterised in that this reaction can be advantageously carried out at ambient temperature or with moderate heating, and, optionally, in a polar solvent, such as methanol, ethanol or propanol.

3. Process according to claim 1, characterised in that the optical isomers of the compounds of the general formula I can be separated after salt-formation using an optically active acid, such as tartaric acid, and fractional crystallisation of the salts, and then, if desired, liberation of the optically active base by means of a strong base or replacement of the acid by a strong acid.

**Patentansprüche** (für die Vertragsstaaten : BE, CH, DE, FR, GB, IT, LI, LU, NL, SE)

1. Verbindungen der allgemeinen Formel

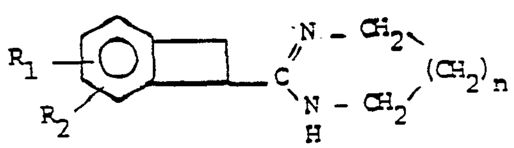

(I)

in der

$R_1$ und $R_2$ jeweils Wasserstoffatome, Halogenatome, Alkylgruppen oder Alkoxygruppen mit 1 bis 4 Kohlenstoffatomen, Sulfonamidgruppen, die gegebenenfalls mit einer Alkylgruppe mit 1 bis 4 Kohlenstoffatomen substituiert sind, oder gemeinsam eine Methylendioxygruppe und

n eine ganze Zahl mit einem Wert von 0 oder 1 bedeuten,

in Form des Racemats oder der optischen Isomeren, sowie deren Additionssalze mit pharmazeutisch annehmbaren Säuren.

2. Verbindungen nach Anspruch 1 der allgemeinen Formel I, in der $R_1$ und $R_2$ jeweils Wasserstoffatome bedeuten.

3. (d,l)-7-(Imidazolin-2-yl)-bicyclo [4.2.0] octatrien-1,3,5 und dessen (d)- und (2)-Isomeren sowie dessen Additionssalze mit pharmazeutisch annehmbaren Säuren.

4. Verfahren zur Herstellung der Verbindungen nach Anspruch 1, dadurch gekennzeichnet, daß man eine Verbindung der allgemeinen Formel

$$H_2N—CH_2—(CH_2)n—CH_2—NH_2 \quad (II)$$

in der n die bezüglich der allgemeinen Formel I angegebenen Bedeutungen besitzt, mit einem Derivat der Formel

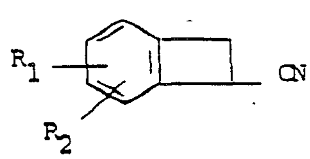

(III)

in der $R_1$ und $R_2$ die bezüglich der Formel I angegebenen Bedeutungen besitzen, in Gegenwart von $H_2S$ bei Raumtemperatur oder unter mäßigem Erhitzen und gegebenenfalls in einem polaren Lösungsmittel kondensiert.

5. Pharmazeutische Zubereitung enthaltend als Wirkstoff eine Verbindung nach einem der Ansprüche 1 bis 3 und ein pharmazeutisch verträgliches Bindemittel oder Trägermaterial.

**Patentansprüche** (für den Vertragsstaat AT)

1. Verfahren zur Herstellung von Bicyclo [4.2.0] octatrien-1,3,5-Derivaten der allgemeinen Formel I

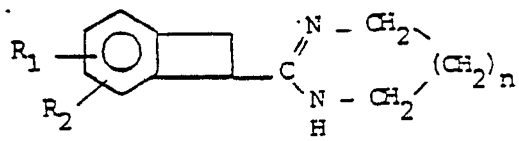

(I)

in der

$R_1$ und $R_2$ jeweils Wasserstoffatome, Halogenatome, Alkygruppen oder Alkoxygruppen mit 1 bis 4 Kohlenstoffatomen, Sulfonamidgruppen, die gegebenenfalls mit einer Alkylgruppe mit 1 bis 4 Kohlenstoffatomen substituiert sind, oder gemeinsam eine Methylendioxygruppe und

n eine ganze Zahl mit einem Wert von 0 oder 1 bedeuten,

in Form des Racemats oder der optischen Isomeren, dadurch gekennzeichnet, daß man ein Derivat der allgemeinen Formel II

$$H_2N—CH_2—(CH_2)n—CH_2—NH_2 \quad (II)$$

in der n die bezüglich der Formel I angegebenen Bedeutungen besitzt, mit einem Derivat der allgemeinen Formel III

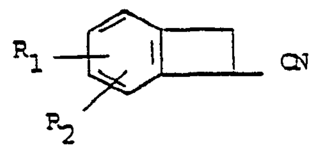

(III)

in der $R_1$ und $R_2$ die bezüglich der Formel I angegebenen Bedeutungen besitzen, in Gegenwart von $H_2S$ kondensiert.

2. Verfahren nach Anspruch 1, dadurch gekennzeichnet, daß die Reaktion mit Vorteil bei Raumtemperatur oder unter mäßigem Erhitzen und gegebenenfalls in einem polaren Lösungsmittel, wie Methanol oder Propanol durchgeführt wird.

3. Verfahren nach Anspruch 1, dadurch gekennzeichnet, daß die optischen Isomeren der Verbindungen der allgemeinen Formel I nach der Salzbildung mit einer optisch aktiven Säure, wie Weinsäure, durch fraktionierte Kristallisation der Salze getrennt werden können, wonach man gewünschtenfalls die optisch aktive Base mit einer starken Base freisetzt oder mit einer starken Säure in ein anderes Salz überführt.